(19) Europäisches Patentamt
European Patent Office
Office européen des brevets

(11) EP 4 326 254 B1

(12) # FASCICULE DE BREVET EUROPEEN

(45) Date de publication et mention de la délivrance du brevet:
**10.06.2026 Bulletin 2026/24**

(21) Numéro de dépôt: **22723667.6**

(22) Date de dépôt: **20.04.2022**

(51) Classification Internationale des Brevets (IPC):
***A61K 31/36*** *(2006.01)* ***A61K 9/00*** *(2006.01)*
***A61K 45/06*** *(2006.01)* ***A61P 3/00*** *(2006.01)*
***A61P 25/08*** *(2006.01)*

(52) Classification Coopérative des Brevets (CPC):
**A61K 31/36; A61K 45/06; A61P 3/00; A61P 25/08**

(86) Numéro de dépôt international:
**PCT/EP2022/060445**

(87) Numéro de publication internationale:
**WO 2022/223636 (27.10.2022 Gazette 2022/43)**

(54) **COMPOSÉS ET COMPOSITIONS POUR LE TRAITEMENT DES HYPERAMMONIÉMIES**

VERBINDUNGEN UND ZUSAMMENSETZUNGEN ZUR BEHANDLUNG VON HYPERAMMONÄMIE

COMPOUNDS AND COMPOSITIONS FOR THE TREATMENT OF HYPERAMMONEMIA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.04.2021 FR 2104119**

(43) Date de publication de la demande:
**28.02.2024 Bulletin 2024/09**

(73) Titulaire: **Biocodex**
**94250 Gentilly (FR)**

(72) Inventeurs:
- **VERLEYE, Marc**
**60190 Remy (FR)**
- **GIRARD, Philippe**
**60280 Margny-lès-Compiègne (FR)**
- **LE GUERN, Marie-Emmanuelle**
**60200 Compiègne (FR)**

(74) Mandataire: **Vial, Lionel et al**
**Cabinet Lionel Vial**
**6 rue de Vaugondran**
**91190 Gif-sur-Yvette (FR)**

(56) Documents cités:
**FR-A1- 2 173 691**

- **CHIRON CATHERINE: "Stiripentol", EXPERT OPINION ON INVESTIGATIONAL DRUGS, INFORMA HEALTHCARE, UK, vol. 14, no. 7, 15 July 2005 (2005-07-15), pages 905 - 911, XP002541844, ISSN: 1354-3784, DOI: 10.1517/13543784.14.7.905**
- **ELAINE C. WIRRELL ET AL: "Stiripentol in Dravet syndrome: Results of a retrospective U.S. study", EPILEPSIA, vol. 54, no. 9, 12 July 2013 (2013-07-12), New York , US, pages 1595 - 1604, XP055368544, ISSN: 0013-9580, DOI: 10.1111/epi.12303**
- **SELVARAJAH ARUNAN ET AL: "A systematic review of adults with Dravet syndrome", SEIZURE, BAILLIERE TINDALL, LONDON, GB, vol. 87, 22 February 2021 (2021-02-22), pages 39 - 45, XP086533114, ISSN: 1059-1311, [retrieved on 20210222], DOI: 10.1016/J.SEIZURE.2021.02.025**
- **ZULFIQAR ALI QURATULAIN ET AL: "Starting stiripentol in adults with Dravet syndrome? Watch for ammonia and carnitine", EPILEPSIA, vol. 61, no. 11, 21 October 2020 (2020-10-21), New York , US, pages 2435 - 2441, XP055862984, ISSN: 0013-9580, DOI: 10.1111/epi.16684**
- **SIMON MATOORI ET AL: "Recent advances in the treatment of hyperammonemia", ADVANCED DRUG DELIVERY REVIEWS, vol. 90, 17 April 2015 (2015-04-17), Amsterdam , NL, pages 55 - 68, XP055335990, ISSN: 0169-409X, DOI: 10.1016/j.addr.2015.04.009**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- RIBAN VERONIQUE ET AL: "Stiripentol inhibits spike-and-wave discharges in animal models of absence seizures: A new mechanism of action involving T-type calcium channels", EPILEPSIA, vol. 63, no. 5, 20 February 2022 (2022-02-20), New York , US, pages 1200 - 1210, XP055944915, ISSN: 0013-9580, DOI: 10.1111/epi.17201

## Description

Domaine de l'invention

La présente demande concerne des composés et des compositions pharmaceutiques utiles dans la prévention ou le traitement de l'hyperammoniémie, notamment de l'hyperammoniémie pharmaco-induite.

Arrière-plan technique

L'hyperammoniémie est un trouble métabolique caractérisé par des niveaux élevés d'ammoniac, un composé contenant de l'azote, dans le sang. L'ammoniac est une source importante d'azote et est nécessaire à la synthèse des acides aminés. Il est également nécessaire pour un équilibre acido-basique normal. Toutefois, lorsqu'il est présent à des concentrations élevées, l'ammoniac est toxique.

Normalement, l'ammoniac est produit dans le côlon et l'intestin grêle d'où il est transporté vers le foie pour être converti via le cycle de l'urée en urée, un composé hydrosoluble qui sera ensuite excrété par les reins.

Les conséquences de l'hyperammoniémie sont variées et peuvent être graves. En effet, l'ammoniac est une neurotoxine puissante et l'entrée de celle-ci dans le cerveau est une cause de troubles neurologiques pouvant conduire à des convulsions, une ataxie, des lésions de type AVC, un coma, une psychose, une perte de vision, une encéphalopathie aiguë, et un œdème cérébral. D'autres symptômes tels que des vomissements, une alcalose respiratoire, une hypothermie et une atteinte hépatique pouvant conduire à la mort sont également observés chez les individus atteints d'hyperammoniémie.

L'hyperammoniémie doit être détectée tôt et traitée immédiatement pour éviter le développement de complications potentiellement mortelles telles qu'un œdème cérébral ou une hernie cérébrale.

Actuellement, la prise en charge de l'hyperammoniémie implique la combinaison de plusieurs mesures afin réduire le taux d'ammoniaque dans le sang. Généralement, les patients reçoivent un régime pauvre en protéine associé à la prise de médicaments destinés à favoriser l'élimination de l'azote, tel que le phénylbutyrate de sodium, le benzoate de sodium et le phénylbutyrate de glycérol (Enns et al., (2007) New Engl J Med., 356 :2282-2292).

Adv. Drug Deliv. Rev. 2015, 90, 55 décrit des produits pour le traitement de l'hyperammoniémie.

Toutefois, ces médicaments doivent être administrés plusieurs fois par jour et engendrent de nombreux effets secondaires, en particulier des nausées, des vomissements, une irritabilité et une anorexie. En outre, en raison de la nécessité de calculer les dosages, un surdosage peut facilement se produire et entraîner des effets secondaires métaboliques graves et la mort.

Ainsi, il subsiste un besoin de traitement et de prévention alternatif efficace de l'hyperammoniémie.

Résumé de l'invention

La présente invention découle de la mise en évidence inattendue par les inventeurs que le stiripentol permettait de diminuer l'hyperammoniémie induite par la méthionine sulfoximine (MSO) ainsi que les convulsions liées à l'hyperammoniémie induite par la méthionine sulfoximine (MSO).

Ainsi, la présente invention concerne un composé de formule (I) suivante :

[Chem 1]

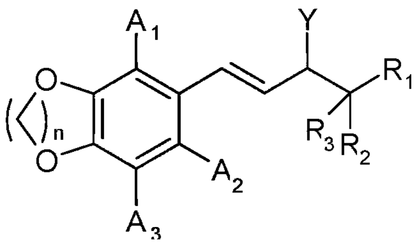

(I)

dans lequel :

- n représente 1 ou 2,
- $A_1$, $A_2$ et $A_3$, identiques ou différents, représentent un atome d'hydrogène, un atome d'halogène, ou un groupe alkyle linéaire ou ramifié ayant 1 à 4 atomes de carbone,

- $R_1$, $R_2$ et $R_3$ représentent indépendamment un atome d'hydrogène ou un groupe alkyle linéaire ou ramifié ayant 1 à 4 atomes de carbone, et
- Y représente -OH, =O ou -SH ;

ou un sel, hydrate ou solvate pharmaceutiquement acceptable de celui-ci,
pour une utilisation dans la prévention ou le traitement de l'hyperammoniémie chez un individu.

La présente invention concerne également le composé de formule (I) ou le sel hydrate ou solvate pharmaceutiquement acceptable de celui-ci pour une utilisation telle que définie ci-dessus en combinaison avec au moins un composé additionnel pour la prévention ou le traitement de l'hyperammoniémie ou pour la prévention ou le traitement d'un ou plusieurs symptômes associés à l'hyperammoniémie.
La présente invention concerne également une composition pharmaceutique comprenant à titre de substance active au moins un composé de formule (I) tel que défini ci-dessus, ou un sel, hydrate ou solvate pharmaceutiquement acceptable de celui-ci, éventuellement en association avec un véhicule pharmaceutiquement acceptable, pour une utilisation dans la prévention ou le traitement de l'hyperammoniémie.
La présente invention concerne également une composition pharmaceutique comprenant à titre de substance active au moins un composé de formule (I) tel que défini ci-dessus, ou un sel, hydrate ou solvate pharmaceutiquement acceptable de celui-ci, éventuellement en association avec un véhicule pharmaceutiquement acceptable, et comprenant optionnellement au moins un composé additionnel pour la prévention ou le traitement de l'hyperammoniémie ou pour la prévention ou le traitement d'un ou plusieurs symptômes associés à l'hyperammoniémie.
La présente invention concerne également une composition pharmaceutique comprenant à titre de substance active au moins un composé de formule (I) tel que défini ci-dessus, ou un sel, hydrate ou solvate pharmaceutiquement acceptable de celui-ci, éventuellement en association avec un véhicule pharmaceutiquement acceptable, et comprenant optionnellement au moins un composé additionnel pour la prévention ou le traitement de l'hyperammoniémie ou pour la prévention ou le traitement d'un ou plusieurs symptômes associés à l'hyperammoniémie sélectionné dans le groupe constitué des médicaments neuroprotecteurs, des médicaments anti-inflammatoires, du benzoate de sodium, du phénylbutyrate de sodium, du phénylbutyrate de glycérol, de la N-carbamylglutamate, du benzoate de sodium, du phénylacétate de sodium, de la lactulose, du chlorhydrate d'arginine, de la L-arginine, de l'acide carglumique, de L-ornithine, de L-aspartate, de la citrulline et de leur mélange.
La présente invention concerne également des produits contenants :

- au moins un composé de formule (I) tel que défini ci-dessus, ou un sel, hydrate ou solvate pharmaceutiquement acceptable de celui-ci, et
- au moins un composé additionnel pour **la** prévention ou le traitement de l'hyperammoniémie ou pour **la** prévention ou le traitement d'un ou plusieurs symptômes associés à l'hyperammoniémie,

comme produit de combinaison pour une utilisation, notamment simultanée, séparée ou étalée dans le temps, pour **la** prévention ou le traitement de l'hyperammoniémie chez un individu.

Description détaillée de l'invention

Comme on l'entend ici, le terme « comprenant » est synonyme « d'incluant », « contenant » ou « englobant » c'est-à-dire que lorsqu'un objet « comprend » une ou plusieurs caractéristiques, d'autres caractéristiques que celles mentionnées peuvent également être comprises dans l'objet. A l'inverse, l'expression « consistant en » signifie « constitué de », c'est-à-dire que lorsqu'un objet « consiste en » une ou plusieurs caractéristiques, l'objet ne peut pas comprendre d'autres caractéristiques que celles mentionnées.

Composé de *formule (I)*

De préférence, le composé de formule (I) tel que défini ci-dessus est représenté par la formule (II) suivante :

[Chem 2]

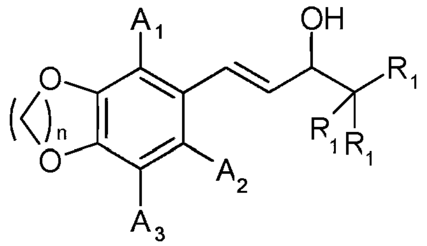

(II)

dans laquelle n, $A_1$, $A_2$, $A_3$ et $R_1$ sont tels que définis ci-dessus.

Plus préférablement, le composé de formule (I) ou (II) défini ci-dessus est représenté par la formule (III) suivante :

[Chem 3]

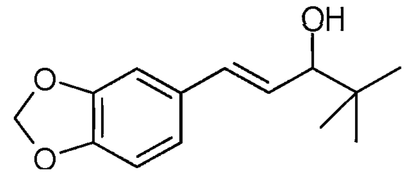

(III)

Les groupes alkyles préférés selon l'invention englobent les groupes méthyle, éthyle, n-propyle, isopropyle, n-butyle, s-butyle et t-butyle.

Les atomes Cl, I, Br ou F sont des atomes d'halogène préférés selon l'invention.

Le brevet français FR 2 173 691 décrit la synthèse du stiripentol, notamment à partir de méthylènedioxy-3,4-phényl-1-diméthyl-4,4-pentén-1-on-3. Il est tout à fait dans les compétences ordinaires de l'homme du métier de synthétiser les autres composés de formule (I) à partir de cet enseignement.

Comme cela apparaîtra clairement à l'homme du métier, les formules (I), (II) et (III) définies ci-dessus représentent soit les divers stéréoisomères englobés par ces formules, soit leurs mélanges, en particulier leurs mélanges racémiques.

Ainsi, le composé de formule (III) peut être un composé de formule (IIIa), un composé de formule (IIIb), ou un mélange d'un composé de formule (IIIa) et d'un composé de formule (IIIb), en particulier le mélange racémique de ceux-ci.

[Chem 3a]

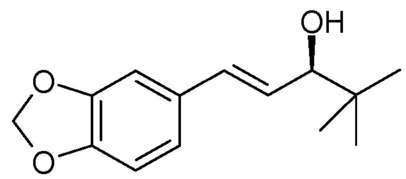

(IIIa)

[Chem 3b]

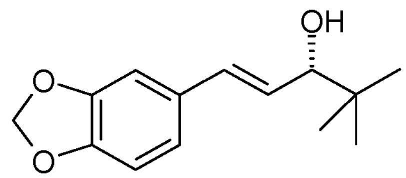

(IIIb)

*Prévention* ou *traitement*

On entend par « hyperammoniémie » l'augmentation des concentrations d'ammoniaque dans le corps. Ainsi l'hyperammoniémie selon l'invention peut être provoquée par exemple par une augmentation de la production d'ammoniaque ou par une diminution des procédés de détoxification de l'ammoniaque dans le corps.

L'ammoniac ($NH_3$), qui peut coexister dans l'organisme avec le cation ammonium ($NH_4^+$) selon le pH, est un produit du catabolisme des protéines et d'autres composés azotés. Il est converti par les enzymes du cycle de l'urée en substance moins toxique, l'urée, avant son excrétion dans l'urine par les reins.

Le cycle de l'urée, qui fonctionne également comme source de production de certains acides aminés (arginine, citrulline et ornithine) dans le corps, comprend 6 enzymes : la carbamoyl phosphate synthase I (CPS), l'ornithine transcarbamylase (OTC), l'argininosuccinate synthétase (ASS), l'argininosuccinate lyase (ASL), l'arginase et la N-acétylglutamate synthase (NAGS) et deux protéines de transport : l'ornithine translocase (ORNT1) et la citrine. Un défaut peut survenir dans l'une ou plusieurs de ces 8 protéines, entrainant une hyperammoniémie, aussi connue sous le nom d'hyperammoniémie primaire.

A titre d'exemple de trouble du cycle de l'urée conduisant à une hyperammoniémie primaire selon l'invention, il est possible de citer :

- un déficit en N-acétylglutamate synthase ;
- un déficit en carbamoylphosphate synthétase 1 ;
- un déficit en ornithine transcarbamylase ;
- un déficit en argininosuccinate synthétase ;
- un déficit en argininosuccinate lyase ;
- un déficit en arginase 1 ;
- le syndrome d'hyperornithinémie-hyperammoniémie-homocitrullinurie ;
- une carence citrulline (citrullinémie de type 2).

L'hyperammoniémie selon l'invention peut également être due à une inhibition du cycle de l'urée à cause d'erreurs du métabolisme intermédiaire caractérisées par une activité réduite des enzymes ou protéines qui ne font pas partie du cycle de l'urée, on parle également d'hyperammoniémie secondaire.

A titre d'exemple d'anomalie provoquant une inhibition du cycle de l'urée conduisant à une hyperammoniémie secondaire selon l'invention, il est possible de citer :

- une acidémie propionique entraînant une carence en glutamate ;
- une acidémies méthylmaloniques conduisant à la production de méthylcitrate ;
- acidémie isovalérique ;
- un déficit en 3-hydroxy-3-méthylglutaryl-CoA-lyase ;
- un déficit en ATP, entraînant un déficit secondaire en CPS1.

- A titre d'exemple d'hyperammoniémie secondaire selon l'invention, il est également possible de citer l'hyperammoniémie causée par une carence en substrats en particulier par :

    - un défauts d'oxydation des acides gras conduisant à une carence en CoA ;
    - des troubles du cycle de la carnitine entraînant un déficit en CoA ;
    - des troubles du complexe pyruvate déshydrogénase conduisant à un déficit en CoA ;
    - un insuffisance hépatique aiguë ou chronique entraînant un déficit en CoA ;
    - une intolérance aux protéines lysinuriques entraînant une carence en citrulline, arginine et ornithine ;
    - le syndrome d'hyperinsulinisme-hyperammoniémie conduisant à une carence en glutamate ;
    - un déficit en pyruvate carboxylase conduisant à un déficit en aspartate ;
    - un déficit en pyrroline-5-carboxylate synthétase conduisant à un déficit en citrulline, arginine et ornithine.

L'hyperammoniémie selon l'invention peut également être liée à ou provoquée par d'autres pathologies notamment les pathologies sélectionnées dans le groupe constitué des maladies du foie, telles qu'une insuffisance hépatique aigue ou chronique, une hépatite virale, une cirrhose, une fonction hépatique altéré, et une dérivation vasculaire du foie entrainant une diminution de la filtration du sang dans le foie. On parle également dans ce cas d'hyperammoniémie acquise.

L'hyperammoniémie selon l'invention peut aussi résulter de la prise de médicament ou être due à une chimiothérapie, on parle d'hyperammoniémie médicamenteuse ou pharmaco-induite. A titre d'exemple de médicament susceptible d'entrainer une hyperammoniémie, il est possible de citer le valproate de sodium, les barbituriques telle que la

primidone, le gel de glycine, la L-asparaginase, le 5-fluoro-uracile, le sunitinib, la chimiothérapie par asparaginase et le regorafenib.

L'hyperammoniémie selon l'invention peut également résulter de la complication d'une transplantation d'organe solide, comme par exemple une transplantation pulmonaire, on parle d'hyperammoniémie post-opérative.

L'hyperammoniémie prévenu ou traiter selon l'invention peut également être l'hyperammoniémie transitoire du nouveau-né.

Dans un mode de réalisation, la présente invention concerne la prévention ou le traitement de troubles associés à l'hyperammoniémie de préférence sélectionnés dans le groupe constitué des convulsions liés à l'hyperammoniémie et des crises convulsives liés à l'hyperammoniémie.

*Individu*

L'individu selon l'invention est de préférence un être humain.

De préférence, l'individu selon l'invention souffre d'hyperammoniémie, en particulier d'hyperammoniémie pharmaco-induite.

Dans un mode de réalisation de l'invention l'individu est un nourrisson né à terme ayant une concentration plasmatique d'ammoniaque d'au moins 40 $\mu$M/L, d'au moins 45 $\mu$M/L, d'au moins 50 $\mu$M/L, d'au moins 80 $\mu$M/L, ou d'au moins 90 $\mu$M/L.

Dans un mode de réalisation de l'invention, l'individu est un nourrisson prématuré ayant une concentration plasmatique d'ammoniaque d'au moins 70 $\mu$M/L, d'au moins 80 $\mu$M/L ou d'au moins 90 $\mu$M/L.

Dans un mode de réalisation de l'invention l'individu est un enfant de plus d'un mois ayant une concentration plasmatique d'ammoniaque d'au moins 40 $\mu$M/L, d'au moins 45 $\mu$M/L, d'au moins 50 $\mu$M/L, d'au moins 80 $\mu$M/L, ou d'au moins 90 $\mu$M/L.

Dans un mode de réalisation de l'invention, l'individu est un adulte ayant une concentration plasmatique d'ammoniaque d'au moins 30 $\mu$M/L, d'au moins 40 $\mu$M/L d'au moins 50 $\mu$M/L, d'au moins 80 $\mu$M/L, d'au moins 150 $\mu$M/L, d'au moins 180 $\mu$M/L, d'au moins 200 $\mu$M/L, d'au moins 300 $\mu$M/L ou d'au moins 400 $\mu$M/L.

Dans un mode de réalisation de l'invention, l'individu selon l'invention présente un trouble du cycle de l'urée.

Dans un mode de réalisation de l'invention, l'individu selon l'invention présentes une perturbation des fonctions cérébrales. De préférence, l'individu selon l'invention présente au moins un trouble sélectionné dans le groupe constitué convulsions, des crises convulsives, des confusions, d'une agitation, des troubles de la conscience, d'une léthargie, d'une ataxie, des lésions de type AVC, d'un coma, une psychose, d'une perte de vision, d'une encéphalopathie aiguë, d'un œdème cérébral, et d'une hernie cérébrale. Plus préférablement, l'individu selon l'invention présente des convulsions ou des crises convulsives

*Administration*

De préférence, le composé de formule (I) tel que défini ci-dessus, ou son sel, hydrate ou solvate pharmaceutiquement acceptable, est administré ou administrable à une dose unitaire, ou est conditionné en dose unitaire, d'environ 50 mg à environ 1500 mg, notamment d'environ 150 mg à 300 mg. De préférence également, le composé de formule (I) tel que définie ci-dessus ou son sel, hydrate ou solvate pharmaceutiquement acceptable est administré ou administrable avec un régime de dosage de 5 g/jour à environ 45g/jour. De préférence, également le composé de formule (I) tel que définie ci-dessus ou son sel, hydrate ou solvate pharmaceutiquement acceptable est administré ou administrable avec un régime de dosage de 50 mg/kg/jour à 500 mg/kg/jour, de préférence de 250 mg/kg/j à 350 mg/kg/j, plus préférablement d'environ 300 mg/kg/j.

De préférence, le composé de formule (I) tel que défini ci-dessus, ou son sel, hydrate ou solvate pharmaceutiquement acceptable, la composition pharmaceutique telle que définie ci-dessus ou le médicament tel que défini ci-dessus est sous une forme acceptable pour une administration par voie orale ou rectale. De préférence, le composé de formule (I) tel que défini ci-dessus, ou son sel, hydrate ou solvate pharmaceutiquement acceptable, la composition pharmaceutique telle que définie ci-dessus ou le médicament tel que défini ci-dessus est administré ou administrable sous la forme d'une poudre, de cachets, de gélules, de sachets ou de suppositoires.

Dans un mode de réalisation, le composé de formule (I) tel que défini ci-dessus, ou son sel, hydrate ou solvate pharmaceutiquement acceptable, la composition pharmaceutique telle que définie ci-dessus ou le médicament tel que défini ci-dessus n'est pas administré avec une autre substance anticonvulsante ou antiépileptique, tels que le valproate de sodium et le clobazam.

Dans un mode de réalisation, le composé de formule (I) tel que défini ci-dessus, ou son sel, hydrate ou solvate pharmaceutiquement acceptable, la composition pharmaceutique telle que définie ci-dessus ou le médicament tel que défini ci-dessus n'est pas administré avec un traitement à la carnitine.

De préférence, le composé additionnel pour la prévention ou le traitement de l'hyperammoniémie ou pour la

prévention ou le traitement d'un ou plusieurs symptômes associés à l'hyperammoniémie selon l'invention est destiné à prévenir ou traiter l'hyperammoniémie, notamment en diminuant la production d'ammoniaque dans le sang ou en augmentant les mécanismes d'élimination de l'ammoniaque dans le sang.

De préférence également, le composé additionnel pour la prévention ou le traitement de l'hyperammoniémie ou pour la prévention ou le traitement d'un ou plusieurs symptômes associés à l'hyperammoniémie peut être tous composés destinés à atténuer ou traiter un ou plusieurs symptômes associés à l'hyperammoniémie, tel que les médicaments neuroprotecteurs ou anti-inflammatoires.

A titre d'exemple de symptômes associés à l'hyperammoniémie, il est possible de citer des vomissements, une perte d'appétit, une alcalose respiratoire, une hypothermie, une atteinte hépatique, des troubles neurologiques tels que des convulsions, des crises convulsives, des confusions, une agitation, des troubles de la conscience, une léthargie, une ataxie, des lésions de type AVC, un coma, une psychose, une perte de vision, une encéphalopathie aiguë, un œdème cérébral, une hernie cérébrale.

De préférence, le composé additionnel utile pour la prévention ou le traitement de l'hyperammoniémie est sélectionné dans le groupe constitué des médicaments neuroprotecteurs tels que la N -acétylcystéine, l'indométacine, le propofol, la minocycline et le mannitol des médicaments anti-inflammatoires, du benzoate de sodium, du phénylbutyrate de sodium, du phénylbutyrate de glycérol, de la N-carbamylglutamate, du benzoate de sodium/phénylacétate de sodium, de la lactulose, du chlorhydrate d'arginine, de la L-arginine, de l'acide carglumique, d'un mélange de L-ornithine et de L-aspartate, de la citrulline, et de leur mélange.

Le composé de formule (I) tel que défini ci-dessus, ou son sel, hydrate ou solvate pharmaceutiquement acceptable, la composition pharmaceutique telle que définie ci-dessus ou le médicament tel que défini ci-dessus peut également être associé à des thérapies destinées à atténuer ou réduire un plusieurs symptômes associés à l'hyperammoniémie ou à prévenir ou traiter l'hyperammoniémie tels que l'hémofiltration, l'hémodialyse intermittente, la dialyse péritonéale, l'hypothermie induite, la thérapie de remplacement rénal.

Le composé de formule (I) tel que défini ci-dessus, ou son sel, hydrate ou solvate pharmaceutiquement acceptable, la composition pharmaceutique telle que définie ci-dessus ou le médicament tel que défini ci-dessus peut également être associé à un régime pauvre en protéine et/ou un régime dans lequel les apports caloriques sont remplacés par du sucre et des lipides.

Comme on l'entend ici, l'expression « combiné » ou « en combinaison » ou « produit de combinaison » signifie que le composé de formule (I) tel que définie ci-dessus, ou un sel, hydrate ou solvate pharmaceutiquement acceptable de celui-ci, en particulier le stiripentol, et le composé additionnel pour la prévention ou le traitement de l'hyperammoniémie ou pour la prévention ou le traitement d'un ou plusieurs symptômes associés à l'hyperammoniémie tel que défini ci-dessus, peuvent être associé au sein d'une même composition pharmaceutique ou d'un même médicament, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée la période de temps pendant laquelle le composé de formule (I) tel que défini ci-dessus ou son sel, hydrate ou solvate pharmaceutiquement acceptable exerce ses effets pharmacologiques sur le l'individu et la période de temps pendant laquelle le composé additionnel tel que défini ci-dessus exerce ses effets pharmacologiques sur le l'individu se recouvrent en totalité ou en partie.

L'invention sera davantage explicitée à l'aide des Exemples et de la Figure non limitatifs qui suivent.

**[Fig.1]**

La Figure 1 représente l'effet du stiripentol (STP) administré par voie intrapéritonéale sur l'ammoniémie (exprimé en μM, axe des ordonnés) après traitement par 50 mg/kg de méthionine sulfoximine (MSO) chez la souris.

* $p < 0.05$ : test statistique ANOVA des groupes traités par rapport au groupe témoin.
\# $p < 0.05$ : test statistique ANOVA des groupes recevant la MSO et le stiripentol par rapport au groupe recevant seulement la MSO.

## EXEMPLE

Les inventeurs ont étudié l'efficacité du stiripentol sur l'hyperammoniémie et sur les convulsions associées à l'hyperammoniémie dans un modèle de convulsions induites par la méthionine sulfoximine (MSO) selon Hevor et al. (1985) Neuropathol. Appl. Neurobiol. 11 : 129-139**,** comme suit.

### A. Matériels et méthodes

**1.** Animaux

Des souris mâles CBA (Janvier) de poids compris entre 23 et 27 grammes sont utilisées après une acclimatation d'au moins 4 jours dans l'animalerie (†° = 22 ± 2°C ; les conditions d'hébergement sont les suivantes : nourriture SAFE "A04" ; eau de boisson : eau du robinet ; cycle nycthéméral : 12 h/12 h (lumière : 7 h/19 h - obscurité : 19 h/7 h)).
A la réception, les souris sont regroupées par 8 à 10 dans des cages 1500U (surface au sol 1500 cm2) avec litière et enrichissement par sizzle dry (SDS), suivant les recommandations éthiques de la Directive 2010/63/UE du Parlement Européen (22/09/2010).

**2.** Protocole

Le jour du test, les souris sont pesées, marqués et mises en cage individuelle. A t = 0 minutes, la MSO (0.1 mL/10 g) est administrée par voie intrapéritonéale, et les souris sont observées attentivement pendant 8 heures. Les animaux sont normaux pendant les quatre premières heures. Des crises toniques et cloniques apparaissent durant quelques heures, puis elles disparaissent progressivement.
Les paramètres évalués sont l'état général des animaux selon la grille d'Irwin (Irwin 1968), le nombres des convulsions, leurs délais d'apparition et la mortalité. Les animaux sont euthanasiés dès l'atteinte des points limites, ou à la fin des 8 heures d'observation, selon la procédure ANI07 approuvée par le Comité d'Ethique.
Des prélèvements de sang et de cerveau entier avec cervelet seront effectués lors de certaines études pour déterminer l'ammoniémie au moment du sacrifice ou au moment de la mort pour les animaux n'allant pas au bout de l'étude.
Le sang est prélevé sous anesthésie à l'isoflurane par ponction cardiaque (0.5 à 1.0 mL par souris) pour déterminer l'ammoniémie. En cas de prélèvement de cerveau et de sang, la souris est sacrifiée rapidement par décapitation : le cerveau est prélevé et congelé dans l'azote liquide. Le foie pourra ensuite être prélevé et congelé rapidement dans l'azote liquide. Les échantillons sont gardés à -80°C.
Le stiripentol (0.1 mL/10g) est administré par voie intrapéritonéale à différents temps par rapport à la MSO.

**3.** Mesure de l'ammoniémie avec le kit ABCAM

Le sang est prélevé après décapitation ou par ponction cardiaque, sous anesthésie à l'isoflurane (débit 1.5 l/mn, évaporateur à 4.3% pour l'induction et le maintien) à l'aide d'une aiguille 25G orange (Terumo, NN2516R) et d'un tube S-Monovette de 1.3 ml contenant de l'EDTA (bouchon mauve, réf 41.1395.105, Sarstedt). Le tube est centrifugé à 2000 g pendant 10 minutes dans la centrifugeuse réfrigérée à 4°C Beckman Allegra. On récupère le plasma dans un microtube de 1 ml (Brand, réf 780500). Les microtubes sont placés dans un congélateur à -80°C avant analyse, sinon sur de la glace pillée.
Le dosage de l'ammoniaque est réalisé par la méthode colorimétrique à l'aide du lecteur de microplaque (Dynex, modèle MRX), et d'un kit ABCAM (réf AB83360, ammonia assay kit) contenant plusieurs réactifs. Ce kit permet une détermination quantitative enzymatique de l'ammonium présent dans les échantillons. Au cours du test, l'ammonium est transformé pour donner un produit qui réagit avec la sonde OxiRed pour une quantification à 570 nm à l'aide d'un lecteur de microplaque. Ce test peut détecter 1 nmol (# 20 μM) d'ammonium. Le kit, gardé à -20°C, comprend les éléments suivants :

- Ammonia assay buffer (25 mL) : prêt à l'emploi, mettre à température ambiante avant utilisation ;
- OxiRed probe (200 μL) : prêt à l'emploi, réchauffer à 37°C pour bien décongeler le DMSO ;
- Developer (1 flacon) : reconstituer avec 220 μL de Ammonia assay buffer ;
- enzyme Mix (1 flacon) : reconstituer avec 220 μL de Ammonia assay buffer;
- Converting Enzyme (1 flacon) : reconstituer avec 220 μL de Ammonia assay buffer ;
- $NH_4Cl$ standard à 10 mM (100 μL) : prêt à l'emploi, à garder sur de la glace pendant l'utilisation.

*Courbe d'étalonnage avec le Standard $NH_4Cl$*

- A réaliser à chaque utilisation ;
- Préparer 100 μL de Standard NH4Cl à 1 mM ;
- 10 μL de Standard $NH_4Cl$ à 10 mM + 90 μL ED ;
- Avec le Standard $NH_4Cl$ à 1 mM, préparer des dilutions selon le tableau 1 suivant :

[Table 1]

Tableau 1 : tableau de dilution

| Standard | Volume de standard (μL) | Assay buffer (μL) | Volume final du standard dans les puits (μL) | Concentration finale d'ammoniaque dans les puits |
|---|---|---|---|---|
| 1 | 0 | 150 | 50 | 0 nmol/puit |
| 2 | 6 | 144 | 50 | 2 nmol/puit |
| 3 | 12 | 138 | 50 | 4 nmol/puit |
| 4 | 18 | 132 | 50 | 6 nmol/puit |
| 5 | 24 | 126 | 50 | 8 nmol/puit |
| 6 | 30 | 120 | 50 | 10 nmol/puit |

Standard à 1 mM soit 1 mmol dans 1 L ou 1 nmol dans 1μL, donc 6 nmol dans 6 μl. En prenant 50 μL sur les 150 μL (6 + 144), on a 2 nmol/puit.

*Préparation des échantillons* :

- Le plasma ou le sérum peuvent être testés directement ;
- Des dilutions à 1/2, 1/5 et 1/10 ;
- Un volume de 5-15 μL de plasma/sérum par puit.

Procédure *pour la mesure* de l'ammoniémie :

- Equilibrer tous les standards/échantillons/matériels à température ambiante ;
- Réaliser chaque mesure en double ;
- Remplissage des puits ;
  - standard : 50 μL de la dilution standard
  - échantillon : 2 à 50 μL de l'échantillon et ajuster à 50 μL avec Assay Buffer
- Préparation du « reaction mix » selon le tableau 2 suivant :

[Table 2]

Tableau 2 : preparation du « reaction mix »

| Composant | Échantillons de mélange réactionnel (μL) |
|---|---|
| Ammonia Assay Buffer | 42 |
| OxiRed Probe | 2 |
| Enzyme Mix | 2 |
| Developer | 2 |
| Converting Enzyme | 2 |

- ajouter 50 μL de reaction mix aux standards et aux échantillons ;
- mélanger et incuber à 37°C pendant 60 minutes en protégeant de la lumière ;
- mesurer à 570 nm.

Calcul *des concentrations* en *ammoniaque*

- Les valeurs trop fortes (en dehors de la gamme standard) doivent être remesurées après dilution ;
- Moyenner des valeurs dupliquées ;
- Soustraire la valeur du « blanc » pour obtenir la DO corrigée ;
- Réaliser une courbe avec les standards pour obtenir l'équation, la pente ;
- Calculer la quantité d'ammonium en nmol/puit (Sa)

$$Sa = (DO\ corrigée - y)\ /pente$$

- Calculer la concentration des échantillons (Sa/Sv) *D avec Sv : volume de l'échantillon ; D : facteur de dilution ; Masse molaire $NH_4^+$ = 18.04 g/mol.

**4.** Produits

La méthionine sulfoximine (Sigma, réf M5379, lots SLBN0115V, SLBP0971V) a été solubilisée dans le NaCl à 0.9%.

Le stiripentol (lot 176) a été mis en suspension dans le Tween 80 à 5%(v/v) et NaCl à 0.9%.

Le kit de dosage ABCAM (réf AB83360, ammonia assay) et $NH_4Cl$ (ammonium chloride, Sigma, réf 254134, masse molaire 53.49 g) sont utilisés pour les dosages de l'ammoniémie.

**5.** Analyse statistique

Les résultats sont exprimés par la valeur moyenne ± ESM. Les tests statistiques utilisés sont :

- Test exact de Fisher pour les pourcentages de convulsions et de mortalité, pour déterminer une différence significative entre les groupes au seuil de 5%,
- Une analyse de variance à un facteur pour les dosages, pour déterminer si l'effet pharmacologique observé est significatif au seuil de 5 % (logiciel SigmaPlot 3.1).

**B. Résultats**

**1.** Effet dose de la methionine sulfoximine

L'administration par voie intrapéritonéale de la méthionine sulfoximine (MSO) a été réalisée à différentes concentrations. Le comportement et la mortalité des souris a été observé jusqu'à 8 heures après l'administration du produit :

- A 40 mg/kg, la MSO entraîne des effets comportementaux modérés : à partir de la 4ème heure de l'hypotonie, de l'hypomotilité, une respiration accélérée, de la ptose et de la mydriase sont observés. En fin d'observation (entre 7 et 8 h), 2 souris sur 10 ont convulsé et l'une d'elle est morte.
- A partir de 50 mg/kg, les signes de toxicité apparaissent dès la 3ème heure. Les convulsions et la mortalité débutent à partir de la 4ème heure après l'injection de la MSO. 80% des souris convulsent avec un taux de 90% de mortalité.
- A 60 mg/kg, la méthionine sulfoximine provoque des convulsions chez 100% des souris, avec un taux de 90% de mortalité.
- A 80 mg/kg, la méthionine sulfoximine provoque des convulsions chez 100% des souris, avec un taux de 100% de mortalité.

Suite à ces résultats, la dose de 50 mg/kg de méthionine sulfoximine a été retenue pour étudier les effets protecteurs du stiripentol sur les troubles induits par la MSO.

2. Effet propre du stiripentol sur l'ammoniémie

Le stiripentol a été administré à 300 mg/kg par voie intrapéritonéale sans administration de méthionine sulfoximine.

Il a été observé que le stiripentol ne modifie par l'ammoniémie (176 ± 14 μM) par rapport au groupe témoin (179 ± 11 μM).

3. Effets du stiripentol après administration de 50 ma/ka de MSO

*2.1. Stiripentol administré juste avant la MSO*

Le stiripentol est administré juste avant la méthionine sulfoximine selon Hevor et al. (1985) Neuropathol. Appl. Neurobiol. 11 : 129-139**.**

L'administration de 50 mg/kg de méthionine sulfoximine a entrainé l'apparition de signes comportementaux de toxicité avec un taux de convulsion de 90% et un taux de mortalité de 75%.

L'administration de 300 mg/kg de stiripentol, juste avant la MSO, a réduit significativement le taux de convulsion à 50% et le taux de mortalité à 25%, suggérant un effet protecteur.

Au cours de cette étude, les dosages de l'ammoniémie ont donné les résultats suivants :

- chez les témoins ne recevant pas la méthionine sulfoximine ni aucun traitement, l'ammoniémie basale est mesurée à 174±18 μM ;
- L'administration de 50 mg/kg de méthionine sulfoximine a entrainé une augmentation significative de l'ammoniémie à 609±51 μM ;
- L'administration de 300 mg/kg de stiripentol, juste avant la MSO, a réduit de 32% cette hyperammoniémie à 417±51 μM.

*2.2. Stiripentol administré 30 minutes avant la MSO*

Le stiripentol est administré par voie intrapéritonéale 30 minutes avant la méthionine sulfoximine selon Cloix et al. (2010). Epilepsia, 51 : 118-128.

L'administration de 50 mg/kg de méthionine sulfoximine a entrainé l'apparition de signes comportementaux de toxicité avec un taux de convulsion de 100% et un taux de mortalité de 80%.

L'administration de 300 mg/kg de stiripentol, 30 minutes avant la MSO, a réduit significativement le taux de convulsion à 45% et le taux de mortalité à 25%, suggérant un effet protecteur.

Au cours de cette étude, les dosages de l'ammoniémie ont donné les résultats suivants (Figure 1) :

- Chez les témoins ne recevant pas la méthionine sulfoximine ni aucun traitement, l'ammoniémie est mesurée à 184 ±12 μM,
- L'administration de 50 mg/kg de méthionine sulfoximine a entrainé une augmentation significative de l'ammoniémie à 763±71 μM,
- L'administration de 300 mg/kg de stiripentol, 30 minutes avant la MSO, a significativement réduit de 41% cette hyperammoniémie à 448±65 μM, par rapport au groupe recevant seulement la MSO.

**C. Conclusion**

Dans le modèle des convulsions induites par la méthionine sulfoximine (MSO), les inventeurs ont montré qu'une dose unique de MSO à 50 mg/kg administrée par voie intrapéritonéale entraîne une hyperammoniémie et des convulsions et de la mortalité chez 80 à 100 % des souris.

Les inventeurs ont montré que l'administration de stiripentol à 300 mg/kg par voie intrapéritonéale diminue significativement les convulsions et la mortalité induites par la méthionine sulfoximine, suggérant un effet protecteur.

La MSO, dont le principal mécanisme d'action est l'inhibition irréversible de la glutamine synthétase, une enzyme impliquée dans la détoxification de l'ammoniaque notamment dans le cerveau, entraîne une hyperammoniémie (Ratnakumari, et al. (1985) J. Neurosci. Res., 14 : 449-59). Dans les astrocytes, la glutamine synthétase utilise l'ammonium et le glutamate pour fabriquer de la glutamine, donc l'inhibition de son activité par la MSO entraîne une augmentation de l'ammoniémie.

Au niveau de l'ammoniémie, les inventeurs ont montré que l'administration de stiripentol à 300 mg/kg par voie intrapéritonéale diminue significativement l'hyperammoniémie induite par la MSO.

**Revendications**

1. Composé de formule (I) suivante :

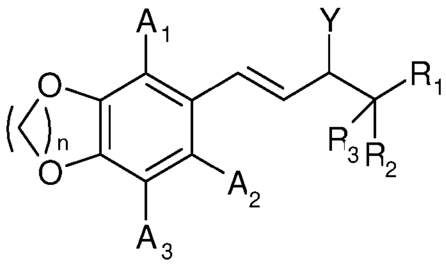

(I)

dans lequel :

- n représente 1 ou 2,
- $A_1$, $A_2$ et $A_3$, identiques ou différents, représentent un atome d'hydrogène, un atome d'halogène, ou un groupe alkyle linéaire ou ramifié ayant 1 à 4 atomes de carbone,
- $R_1$, $R_2$ et $R_3$ représentent indépendamment un atome d'hydrogène ou un groupe alkyle linéaire ou ramifié ayant 1 à 4 atomes de carbone, et
- Y représente -OH, =O ou -SH ;

ou un sel, hydrate ou solvate pharmaceutiquement acceptable de celui-ci,
pour une utilisation dans la prévention ou le traitement de l'hyperammoniémie chez un individu.

2. Composé ou sel, hydrate ou solvate pharmaceutiquement acceptable de celui-ci pour une utilisation selon la revendication 1, dans lequel le composé de formule (I) est de formule (II) suivante :

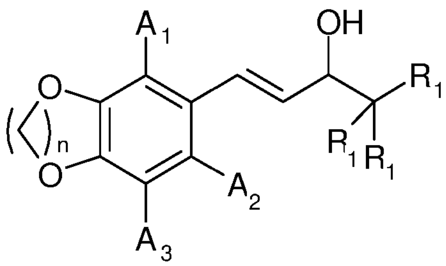

(II)

dans lequel n, A1, A2, A3 et $R_1$ sont tels que définis dans la revendication 1.

3. Composé ou sel, hydrate ou solvate pharmaceutiquement acceptable de celui-ci pour une utilisation selon la revendication 1 ou 2, dans lequel le composé de formule (I) est de formule (III) suivante :

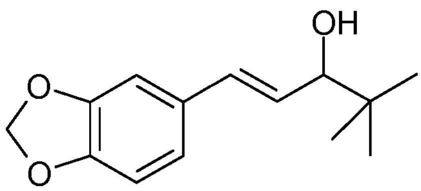

(III).

4. Composé ou sel, hydrate ou solvate pharmaceutiquement acceptable de celui-ci pour une utilisation selon l'une quelconque des revendications 1 à 3, pour la prévention ou le traitement des convulsions liés à l'hyperammoniémie.

5. Composé ou sel, hydrate ou solvate pharmaceutiquement acceptable de celui-ci pour une utilisation selon l'une quelconque des revendications 1 à 4, pour la prévention ou le traitement de l'hyperammoniémie pharmaco-induite.

6. Composé ou sel, hydrate ou solvate pharmaceutiquement acceptable de celui-ci pour une utilisation selon l'une quelconque des revendications 1 à 5, conditionné en une dose unitaire de 50mg à 1500 mg.

7. Composé ou sel, hydrate ou solvate pharmaceutiquement acceptable de celui-ci pour une utilisation selon l'une quelconque des revendications 1 à 6, convenant pour une administration par voie orale.

8. Composé ou sel, hydrate ou solvate pharmaceutiquement acceptable de celui-ci pour une utilisation selon l'une quelconque des revendications 1 à 7, sous forme d'une poudre, de cachets, de gélules, de sachets ou de suppositoires.

9. Composé ou sel, hydrate ou solvate pharmaceutiquement acceptable de celui-ci pour une utilisation selon l'une quelconque des revendications 1 à 8, en combinaison avec au moins un composé additionnel pour la prévention ou le traitement de l'hyperammoniémie ou pour la prévention ou le traitement d'un ou plusieurs symptômes associés à l'hyperammoniémie.

10. Composé ou sel, hydrate ou solvate pharmaceutiquement acceptable de celui-ci pour une utilisation selon l'une quelconque des revendications 1 à 9, en combinaison avec au moins un composé additionnel pour la prévention et le traitement de l'hyperammoniémie ou pour la prévention ou le traitement d'un ou plusieurs symptômes associés à l'hyperammoniémie, sélectionné dans le groupe constitué des médicaments neuroprotecteurs, des médicaments anti-inflammatoires, du benzoate de sodium, du phénylbutyrate de sodium, du phénylbutyrate de glycérol, de la N-carbamylglutamate, du benzoate de sodium, du phénylacétate de sodium, de la lactulose, du chlorhydrate d'arginine, de la L-arginine, de l'acide carglumique, de L-ornithine, de L-aspartate, de la citrulline et de leur mélange.

11. Composition pharmaceutique comprenant à titre de substance active au moins un composé de formule (I) tel que défini dans l'une quelconque des revendication 1 à 3, ou un sel, hydrate ou solvate de celui-ci, éventuellement en association avec un véhicule pharmaceutiquement acceptable, pour une utilisation selon l'une quelconque des revendications 1, 4 et 5, et comprenant optionnellement au moins un composé additionnel pour la prévention ou le traitement de l'hyperammoniémie ou pour la prévention ou le traitement d'un ou plusieurs symptômes associés à l'hyperammoniémie.

12. Composition pharmaceutique comprenant à titre de substance active au moins un composé de formule (I) tel que défini dans l'une quelconque des revendication 1 à 3, ou un sel, hydrate ou solvate de celui-ci, éventuellement en association avec un véhicule pharmaceutiquement acceptable, pour une utilisation selon l'une quelconque des revendications 1, 4 et 5, et comprenant au moins un composé additionnel pour la prévention ou le traitement de l'hyperammoniémie ou pour la prévention ou le traitement d'un ou plusieurs symptômes associés à l'hyperammoniémie sélectionné dans le groupe constitué des médicaments neuroprotecteurs, des médicaments anti-inflammatoires, du benzoate de sodium, du phénylbutyrate de sodium, du phénylbutyrate de glycérol, de la N-carbamylglutamate, du benzoate de sodium, du phénylacétate de sodium, de la lactulose, du chlorhydrate d'arginine, de la L-arginine, de l'acide carglumique, de L-ornithine, de L-aspartate, de la citrulline et de leur mélange.

13. Produits contenant :

   - au moins un composé de formule (I) tel que défini dans l'une des revendications 1 à 3, ou un sel, hydrate ou solvate pharmaceutiquement acceptable de celui-ci, et
   - au moins un composé additionnel pour la prévention ou le traitement de l'hyperammoniémie ou pour la prévention ou le traitement d'un ou plusieurs symptômes associés à l'hyperammoniémie,

   comme produit de combinaison pour une utilisation, notamment simultanée, séparée ou étalée dans le temps, pour la prévention ou le traitement de l'hyperammoniémies chez un individu.

## Patentansprüche

1. Verbindung der folgenden Formel (I):

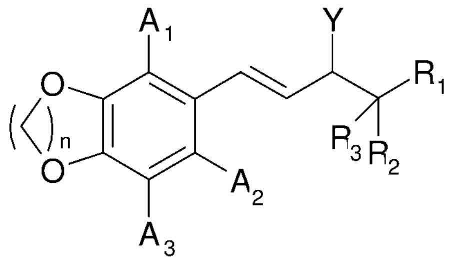

(I)

in der

- n für 1 oder 2 steht,
- $A_1$, $A_2$ und $A_3$, identisch oder verschieden, für ein Wasserstoffatom, ein Halogenatom oder eine lineare oder verzweigte Alkyl-Gruppe mit 1 bis 4 Kohlenstoffatomen steht,
- $R_1$, $R_2$ und $R_3$ unabhängig voneinander für ein Wasserstoffatom oder eine lineare oder verzweigte Alkyl-Gruppe mit 1 bis 4 Kohlenstoffatomen steht, und
- Y für -OH, =O oder -SH steht,

oder ein pharmazeutisch verträgliches Salz, Hydrat oder Solvat davon,
zum Gebrauch in der Vorbeugung oder der Behandlung von Hyperammonämie bei einem Patienten.

**2.** Verbindung oder pharmazeutisch verträgliches Salz, Hydrat oder Solvat davon zum Gebrauch nach Patentanspruch 1, in der die Verbindung der Formel (I) der folgenden Formel (II) genügt:

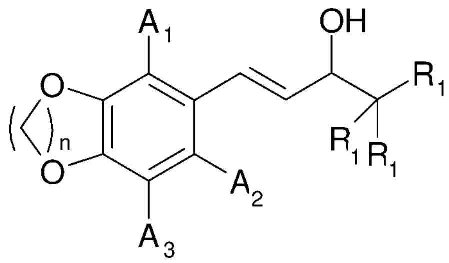

(II)

in der n, $A_1$, $A_2$, $A_3$, und $R_1$ wie in Patentanspruch 1 definiert sind.

**3.** Verbindung oder pharmazeutisch verträgliches Salz, Hydrat oder Solvat davon zum Gebrauch nach Patentanspruch 1 oder 2, in der die Verbindung der Formel (I) der folgenden Formel (III) genügt:

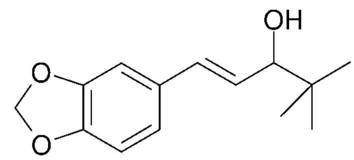

(III)

**4.** Verbindung oder pharmazeutisch verträgliches Salz, Hydrat oder Solvat davon zum Gebrauch nach irgendeinem der Patentansprüche 1 bis 3 zur Vorbeugung oder Behandlung mit Hyperammonämie verbundener Konvulsionen.

**5.** Verbindung oder pharmazeutisch verträgliches Salz, Hydrat oder Solvat davon zum Gebrauch nach irgendeinem der Patentansprüche 1 bis 4 zur Vorbeugung oder Behandlung pharmakogener Hyperammonämie.

**6.** Verbindung oder pharmazeutisch verträgliches Salz, Hydrat oder Solvat davon zum Gebrauch nach irgendeinem der Patentansprüche 1 bis 5, in Einzelgaben von 50 mg bis 1500 mg portioniert.

7. Verbindung oder pharmazeutisch verträgliches Salz, Hydrat oder Solvat davon zum Gebrauch nach irgendeinem der Patentansprüche 1 bis 6, in einer zur oralen Verabreichung geeigneten Form.

8. Verbindung oder pharmazeutisch verträgliches Salz, Hydrat oder Solvat davon zum Gebrauch nach irgendeinem der Patentansprüche 1 bis 7, in Form eines Pulvers, von Tabletten, Gelatinekapseln, Tüten oder Zäpfchen.

9. Verbindung oder pharmazeutisch verträgliches Salz, Hydrat oder Solvat davon zum Gebrauch nach irgendeinem der Patentansprüche 1 bis 8, in Kombination mit mindestens einer zusätzlichen Verbindung zur Vorbeugung oder Behandlung von Hyperammonämie oder zur Vorbeugung oder Behandlung eines oder mehrerer mit Hyperammonämie verbundener Symptome.

10. Verbindung oder pharmazeutisch verträgliches Salz, Hydrat oder Solvat davon zum Gebrauch nach irgendeinem der Patentansprüche 1 bis 9, in Kombination mit mindestens einer zusätzlichen Verbindung zur Vorbeugung oder Behandlung von Hyperammonämie oder zur Vorbeugung oder Behandlung eines oder mehrerer mit Hyperammonämie verbundener Symptome, gewählt aus der Gruppe, bestehend aus neuroprotektiven Medikamenten, entzündungshemmenden Medikamenten, Natriumbenzoat, Natriumphenylbutyrat, Glycerolphenylbutyrat, N-Carbamylglutamat, Natriumphenylazetat, Laktulose, Argininhydrochlorid, L-Arginin, Carglumsäure, L-Ornithin, L-Asparaginsäure, Citrullin und ihren Mischungen.

11. Pharmazeutische Zubereitung, als Wirkstoff mindestens eine Verbindung der Formel (I) nach irgendeinem der Patentansprüche 1 bis 3 oder ein pharmazeutisch verträgliches Salz, Hydrat oder Solvat davon enthaltend, eventuell in Kombination mit einem pharmazeutisch verträglichen Vehikel, zum Gebrauch nach irgendeinem der Patentansprüche 1, 4 und 5, und optional mindestens eine zusätzliche Verbindung zur Vorbeugung oder Behandlung von Hyperammonämie oder zur Vorbeugung oder Behandlung eines oder mehrerer mit Hyperammonämie verbundener Symptome enthaltend.

12. Pharmazeutische Zubereitung, als Wirkstoff mindestens eine Verbindung der Formel (I) nach irgendeinem der Patentansprüche 1 bis 3 oder ein pharmazeutisch verträgliches Salz, Hydrat oder Solvat davon enthaltend, eventuell in Kombination mit einem pharmazeutisch verträglichen Vehikel, zum Gebrauch nach irgendeinem der Patentansprüche 1, 4 und 5, und optional mindestens eine zusätzliche Verbindung zur Vorbeugung oder Behandlung von Hyperammonämie oder zur Vorbeugung oder Behandlung eines oder mehrerer mit Hyperammonämie verbundener Symptome enthaltend, gewählt aus der Gruppe, bestehend aus neuroprotektiven Medikamenten, entzündungshemmenden Medikamenten, Natriumbenzoat, Natriumphenylbutyrat, Glycerolphenylbutyrat, N-Carbamylglutamat, Natriumphenylazetat, Laktulose, Argininhydrochlorid, L-Arginin, Carglumsäure, L-Ornithin, L-Asparaginsäure, Citrullin und ihren Mischungen.

13. Produkt, enthaltend:

- mindestens eine Verbindung der Formel (I) nach irgendeinem der Patentansprüche 1 bis 3 oder ein pharmazeutisch verträgliches Salz, Hydrat oder Solvat davon, und
- mindestens eine zusätzliche Verbindung zur Vorbeugung oder Behandlung von Hyperammonämie oder zur Vorbeugung oder Behandlung eines oder mehrerer mit Hyperammonämie verbundener Symptome,

als Kombinationsprodukt zum insbesondere gleichzeitigen, getrennten oder zeitlich versetzten Gebrauch zur Vorbeugung oder Behandlung von Hyperammonämie bei einem Patienten.

## Claims

1. A compound of the following formula (I)

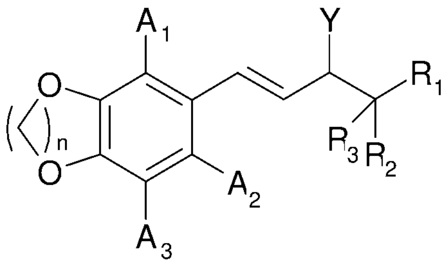

(I)

wherein:

- n represents 1 or 2,
- $A_1$, $A_2$, and $A_3$, which may be identical or different, represent a hydrogen atom, a halogen atom, or a linear or branched alkyl group having 1 to 4 carbon atoms,
- $R_1$, $R_2$, and $R_3$ independently represent a hydrogen atom or a linear or branched alkyl group having 1 to 4 carbon atoms, and
- Y represents -OH, =O or -SH;

or a pharmaceutically acceptable salt, hydrate or solvate thereof,
for use in the prevention or treatment of hyperammonemia in an individual.

**2.** The compound or pharmaceutically acceptable salt, hydrate, or solvate thereof for use according to claim 1, wherein the compound of formula (I) is of the following formula (II):

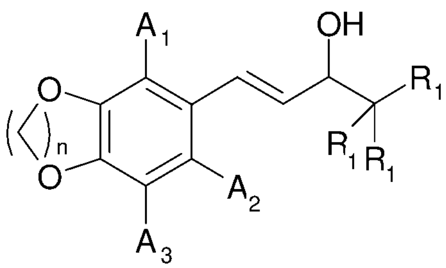

(II)

wherein n, A1, A2, A3, and $R_1$ are as defined in claim 1.

**3.** The compound or pharmaceutically acceptable salt, hydrate, or solvate thereof for use according to claim 1 or 2, wherein the compound of formula (I) is of the following formula (III):

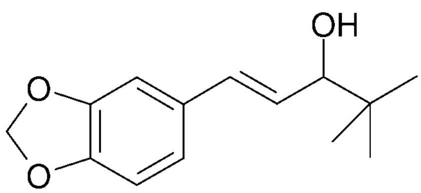

(III).

**4.** The compound or pharmaceutically acceptable salt, hydrate, or solvate thereof, for use according to any one of claims 1 to 3, for the prevention or treatment of seizures related to hyperammonemia.

**5.** The compound or pharmaceutically acceptable salt, hydrate, or solvate thereof, for use according to any one of claims 1 to 4, for the prevention or treatment of drug-induced hyperammonemia.

**6.** The compound or pharmaceutically acceptable salt, hydrate, or solvate thereof, for use according to any one of claims 1 to 5, packaged in a unit dose of 50 mg to 1500 mg.

**7.** The compound or pharmaceutically acceptable salt, hydrate, or solvate thereof, for use according to any one of claims 1 to 6, suitable for oral administration.

**8.** The compound or pharmaceutically acceptable salt, hydrate, or solvate thereof, for use according to any one of claims 1 to 7, in the form of a powder, tablets, capsules, sachets, or suppositories.

**9.** The compound or pharmaceutically acceptable salt, hydrate, or solvate thereof, for use according to any one of claims 1 to 8, in combination with at least one additional compound for the prevention or treatment of hyperammonemia or for the prevention or treatment of one or more symptoms associated with hyperammonemia.

**10.** The compound or pharmaceutically acceptable salt, hydrate, or solvate thereof, for use according to any one of claims 1 to 9, in combination with at least one additional compound for the prevention or treatment of hyperammonemia or for the prevention or treatment of one or more symptoms associated with hyperammonemia selected from the group consisting of neuroprotective drugs, anti-inflammatory drugs, sodium benzoate, sodium phenylbutyrate, glycerol phenylbutyrate, N-carbamylglutamate, sodium benzoate, sodium phenylacetate, lactulose, arginine hydrochloride, L-arginine, carglumic acid, L-ornithine, L-aspartate, citrulline, and mixtures thereof.

**11.** A pharmaceutical composition comprising as active substance at least one compound of formula (I) as defined in any one of claims 1 to 3, or a salt, hydrate, or solvate thereof, optionally in combination with a pharmaceutically acceptable carrier, for use as defined in any one of claims 1, 4 and 5, and optionally comprising at least one additional compound for the prevention or treatment of hyperammonemia or for the prevention or treatment of one or more symptoms associated with hyperammonemia.

**12.** A pharmaceutical composition comprising as active substance at least one compound of formula (I) as defined in any of claims 1 to 3, or a salt, hydrate or solvate thereof, optionally in combination with a pharmaceutically acceptable carrier, for use as defined in any one of claims 1, 4 and 5, and comprising at least one additional compound for the prevention or treatment of hyperammonemia or for the prevention or treatment of one or more symptoms associated with hyperammonemia selected from the group consisting of neuroprotective drugs, anti-inflammatory drugs, sodium benzoate, sodium phenylbutyrate, glycerol phenylbutyrate, N-carbamylglutamate, sodium benzoate, sodium phenylacetate, lactulose, arginine hydrochloride, L-arginine, carglumic acid, L-ornithine, L-aspartate, citrulline, and mixtures thereof.

**13.** Product containing:

- at least one compound of formula (I) as defined in any of claims 1 to 3, or a pharmaceutically acceptable salt, hydrate, or solvate thereof, and
- at least one additional compound for the prevention or treatment of hyperammonemia or for the prevention or treatment of one or more symptoms associated with hyperammonemia,

as a combination product for use, in particular simultaneous, separate, or staggered over time, for the prevention or treatment of hyperammonemia in an individual.

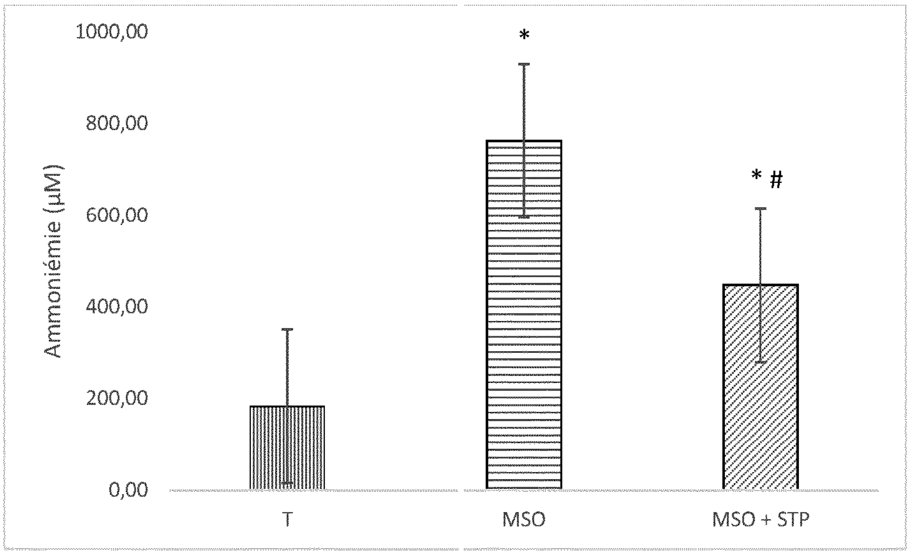
1000,00
800,00
600,00
400,00
200,00
0,00
Ammoniémie (µM)
*
* #
T
MSO
MSO + STP

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2173691


### Littérature non-brevet citée dans la description


- **ENNS et al.** *New Engl J Med.*, 2007, vol. 356, 2282-2292
- *Adv. Drug Deliv. Rev.*, 2015, vol. 90, 55
- **HEVOR et al.** *Neuropathol. Appl. Neurobiol.*, 1985, vol. 11, 129-139
- **CLOIX et al.** *Epilepsia*, 2010, vol. 51, 118-128
- **RATNAKUMARI et al.** *J. Neurosci. Res.*, 1985, vol. 14, 449-59